# EUROPEAN PATENT APPLICATION

(11) **EP 4 342 449 A1**
(43) Date of publication of application: **27.03.2024**
(21) Application number: 22196979.3
(22) Date of filing: 21.09.2022
(51) Int. Cl.: A61K 9/00, A61K 9/06, A61K 47/10, A61K 31/196, A61P 29/00

(54) **HYDROALCOHOLIC SINGLE PHASE GEL COMPOSITIONS FOR TOPICAL DELIVERY OF DICLOFENAC**

(71) Applicant: Haleon CH SARL, 1197 Prangins (CH)
(72) Inventor: KISAK, Ed, San Diego (US); LU, Guojin, Richmond (US); NEWSAM, John, San Diego (US); PATIL, Shantesh, Richmond (US)
(74) Representative: Haleon Patent Department

(57) **Abstract**

Hydroalcoholic single phase gel compositions comprising diclofenac or a pharmaceutically acceptable salt of diclofenac are disclosed. The compositions are single phase compositions, and don't contain a lipophilic phase. The compositions can comprise at least one non-ionic surfactant. The compositions are useful in the topical delivery of diclofenac. The compositions can be used in the treatment of for example pain.

## Description

### FIELD OF THE INVENTION

This disclosure relates to formulations for the topical delivery of diclofenac. Specifically, it relates to formulations which are hydroalcoholic gels which are non-greasy.

### BACKGROUND TO THE INVENTION

Diclofenac is a non-steroidal anti-inflammatory drug (NSAID) of the acetic acid class widely used in systemic and in topical or transdermal products. Emulsion gels, also called emulgels, are oil-in-water emulsions with a gelled aqueous phase. Emulgels for diclofenac topical delivery are available on the market. Recent patient reports suggest that some users are dissatisfied with some of the attributes of emulgels. The present inventors have overcome those disadvantages, and the present disclosure provides a topical diclofenac product that has advantages similar to the existing products, but additionally has attributes that satisfy the evolving consumer preferences.

### SUMMARY OF THE INVENTION

There is disclosed a hydroalcoholic single phase gel composition for topical delivery of diclofenac, comprising:
- diclofenac or a pharmaceutically acceptable salt of diclofenac,
- a gelling agent,
- aliphatic C₂-C₄ mono-alcohol in a concentration of about 5% w/w to about 20% w/w,
- aliphatic diol in a concentration of about 2.5% w/w to about 20% w/w,
- a non-ionic emulsifier, and
- water.

In one embodiment, the composition comprises diclofenac or a pharmaceutically acceptable salt of diclofenac in a total concentration of about 1.5% w/w to about 2.5% w/w diclofenac sodium equivalent.

In one embodiment, the composition comprises at least two different non-ionic emulsifiers.

In one embodiment, the composition comprises a non-ionic emulsifier with a hydrophilic-lipophilic balance value of about 13 to about 17, about 14 to about 16, or about 15.

In one embodiment, the composition comprises a non-ionic emulsifier with a hydrophilic-lipophilic balance value of about 15 to about 19, about 16 to about 18, or about 17.

In one embodiment, the composition comprises a non-ionic emulsifier selected from the group consisting of Macrogol Cetostearyl Ether 20, Polysorbate 60, Polysorbate 80, Isosteareth 20, PEG-60 Almond Glycerides, PEG-20 Methyl Glucose Sesquistearate, Oleth-20, Steareth-20, and combinations of two or more thereof.

In one embodiment, the composition comprises a macrogol cetostearyl ether.

In one embodiment, the composition comprises a non-ionic emulsifier selected from the group of Polysorbate 20, Laureth-20, and combinations thereof.

In one embodiment, the composition comprises Polysorbate 20.

In one embodiment, the composition comprises a non-ionic emulsifier comprising a 2-isopropyl-tetrahydrofuran moiety comprising one or more oligo(ethylene oxide) ester group, wherein all ethylene oxide units of the oligo(ethylene oxide) ester groups in combination comprise 10 to 30, 14 to 24, or 20 carbon atoms.

In one embodiment, the composition comprises a first non-ionic emulsifier with a hydrophilic-lipophilic balance value of about 14 to about 16, and a second non-ionic emulsifier with a hydrophilic-lipophilic balance value of about 16 to about 18.

In one embodiment, the composition comprises a first non-ionic emulsifier which is a macrogol cetostearyl ether and a second non-ionic emulsifier which is a polysorbate.

In one embodiment, the composition comprises non-ionic emulsifier in a total concentration of about 0.1% w/w to about 5% w/w, about 1% w/w to about 3.5% w/w or about 1.5% w/w to about 3% w/w.

In one embodiment, the composition comprises isopropanol in a concentration of about 8% w/w to about 15% w/w.

In one embodiment, the composition comprises propylene glycol in a concentration of about 3% w/w to about 15% w/w.

In one embodiment, the non-ionic emulsifier retards permeation of diclofenac.

In one embodiment, the composition is substantialla free of one or more of a fat, an oil, a wax, an occlusive agent, or carbohydrate-based ointment base.

In one embodiment, the composition is substantially free of fat, oil, wax, occlusive agent, and carbohydrate-based ointment base.

In one embodiment, the composition is substantially free from oils.

In one embodiment, the composition is substantially free from fats.

In one embodiment, the composition is substantially free from waxes.

In one embodiment, the composition is substantially free from carbohydrate-based ointment base.

In one embodiment, the composition is substantially free form a lipophilic dispersed phase.

In one embodiment, the gel is transparent.

In one embodiment, the composition has a pH of about 6.5 to about 8.5.

In one embodiment, the composition has a cumulative permeation in an *in vitro* skin permeation test, that enables to rely on literature data of Voltarol Joint Pain Relief 2.32% diclofenac diethylammonium, for a registration under the well-established use criteria, according to Annex I of Directive 2001/83/EC. There are also disclosed the compositions for use in a method of treatment of pain of non-serious arthritis of the knee or fingers.

There is also disclosed the use of a non-ionic emulsifier to reduce cumulative permeation of diclofenac through the skin over a period of 6 hours, 9 hours, 12 hours or 24 hours, from a hydroalcoholic single phase gel for topical delivery of diclofenac.

### DESCRIPTION OF DRAWINGS/FIGURES

FIG. 1: An example of a hydroalcoholic single phase gel composition, when used with a dosing card.
FIG 2: Visual representation of the results of Example 2
FIG. 3: Visual representation of the results of Example 3

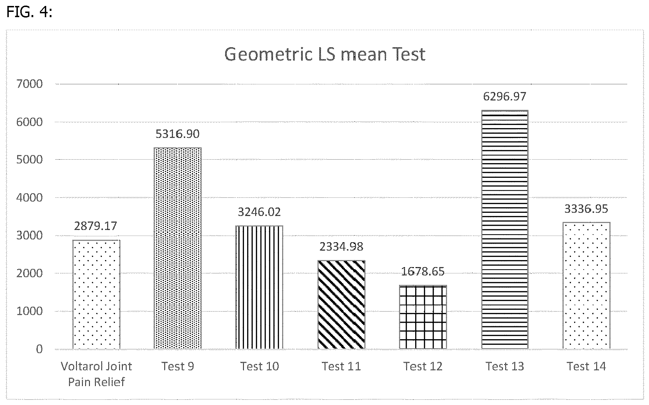

FIG. 4: Visual representation of the results of Example 4

### DETAILED DESCRIPTION OF THE INVENTION

An example of a topical diclofenac formulation is Voltaren Arthritis Pain (diclofenac sodium topical gel, 1% (NSAID)- arthritis pain reliever) which comprises 1% w/w diclofenac sodium and is approved in the USA as an over the counter (OTC) drug since 2020. Another topical diclofenac formulation comprises 1.16% w/w diclofenac diethylammonium salt (equivalent to 1% of the diclofenac sodium salt), sold as Voltaren or Voltarol 1.16% Emulgel. Voltaren 1.16% Emulgel has been marketed in Switzerland since 1974. In some countries, for example in the United Kingdom, Voltarol Joint Pain Relief 2.32% Gel is available on the market which contains 2.32% w/w Diclofenac Diethylammonium (DEA) salt (2% w/w diclofenac sodium equivalent). Voltaren Arthritis Pain, Voltaren 1.16% Emulgel, and Voltarol Joint Pain Relief are emulsion gels (emulgels). Emulgels are a particular format for topical application of drugs. The emulgel format has been described in combination with the active ingredient (API) diclofenac e.g. in US4917886 A.

According to US4917886 A, the described emulgel formulations provide a readier solubility of the active ingredient, and an associated higher effective ingredient concentration compared to conventional topical formulations such as gels or creams. Another advantageous property of the emulgels is described to be the presence of a lipid phase which provides fat-restoring properties. The combination of a lipid phase with a gelled aqueous phase enables the formulation to be massaged in whilst, at the same time, the direct absorption into the skin is experienced as a pleasant property.

Existing Voltaren 1%, 1.16% and 2.32% emulgel products are successful commercial products. However, due to evolving consumer preference over time, it has become an objective to identify new compositions that are absorbed quickly into the skin without leaving oily traces or residues on the application site and on the hands, as well as being optically transparent or clear.

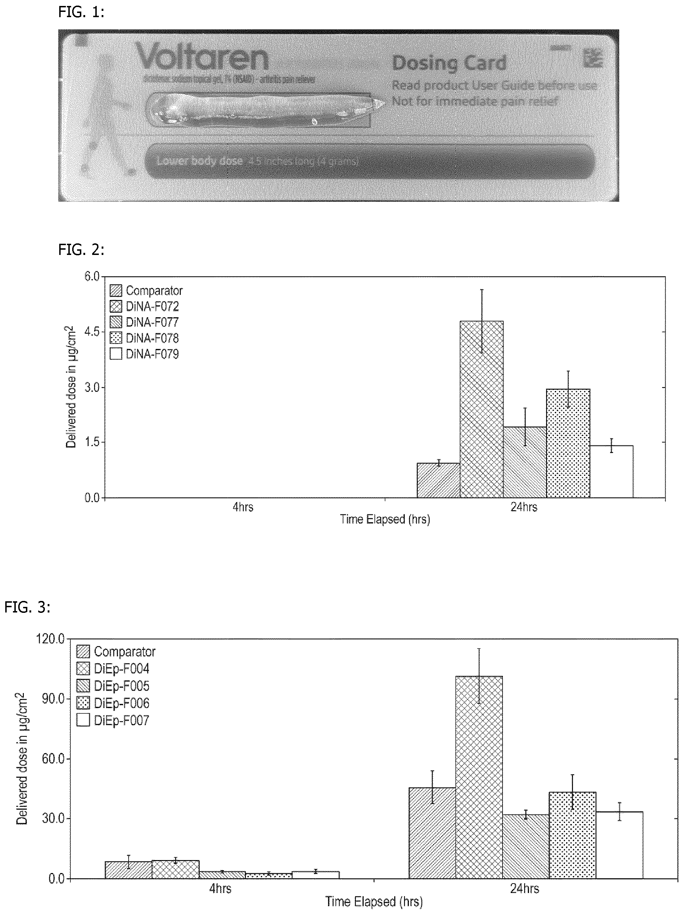

It was a main objective for the inventors to find new compositions that are optically transparent, or clear like water. Transparent compositions may be recognised as modern, clean, fresh, and natural. This may increase user compliance. Moreover, clear topical products may be easier to dose for some users. For example, on a conventional dosing card for topical products, a transparent gel does not occlude a scale or measurement area (see Fig. 1).

At the same time, advantageous properties existing Voltaren 1%, 1.16% or 2.32% emulgel products should not be compromised. The new compositions should comprise a similar diclofenac dose. They should provide a similar diclofenac permeation profile. By that, a similar efficacy and safety could be expected. Moreover, beneficial sensory attributes of the existing products should be maintained or improved, such as pleasant spreading experience and a cooling effect.

For topical compositions comprising diclofenac or diclofenac salts, it can be challenging to fully and permanently solubilise the diclofenac (salt). For example, the diclofenac free acid is a weak acid and poorly soluble in water. For topical compositions comprising diclofenac (salt), crystallisation or precipitation of diclofenac (salt) during storage and after application to the skin is a concern, because it could lead to dosing inaccuracies or underdosing, and to "loss" of API, as unsolubilsed API is no longer available for permeation through the skin. Therefore, a solvent system is required that fully solubilises diclofenac or its salts. In the art, solvent systems comprising water, an aliphatic C₂-C₄ mono-alcohol and an aliphatic diol have been used. For example, Voltarol 2.32% Emulgel comprises such a combination of solvents. However, hydroalcoholic gels comprising the solvent systems of the art can't achieve the objectives described above. Even if solubilisation of diclofenac or diclofenac salts was achieved, other attributes of the gels can go haywire. For example, skin permeation of diclofenac can be impacted. Aliphatic C₂-C₄ mono-alcohols can have a permeation enhancing effect. The effect of the aliphatic diols on permeation rate (enhancing or reducing) can depend on their total concentration. Higher concentrations of aliphatic diol can furthermore render compositions hazy or turbid, which would not be acceptable for the present inventors. Moreover, other excipients in a given formulation can also impact skin permeation. US4917886 A, cited above, describes emulgels comprising a lipophilic phase as an option to enhance API solubilisation. But as emulgels are typically opaque, and the present inventors sought to find transparent compositions, a different format was required. The present inventors observed (see Example 1) that hydroalcoholic gel compositions consisting of the hydroalcoholic phase of Voltaren 1.16% emulgel showed sufficient API solubilisation. However, their cumulative permeation exceeded that of Voltaren 1.16% emulgel to a degree that they did not meet the defined permeation characteristic requirements. The inventors thus found that hydroalcoholic single phase gel compositions, in comparison to emulgels, tend to have a higher permeation rate of the API. Without being bound to a specific theory, the inventors believe that this is due to the lack of lipophilic dispersed phase, and a higher water activity of compositions comprising high concentrations of water. However, the inventors aimed at formulating compositions with permeation characteristics similar to those of the existing Voltaren 1% or 1.16% Emulgel products. By that, the posology, i.e. the dosage amounts and frequencies, of the existing products can be maintained. It was therefore a particular challenge to identify suitable solvents and their useful concentrations that fully solubilise the diclofenac or diclofenac salt in a single phase hydroalcoholic composition, which can form transparent gels that have the desired application properties, and that have an acceptable (not too high) cumulative permeation.

Surprisingly, the present inventors were able to formulate a hydroalcoholic single phase gel with permeation properties very similar to those of emulgels.

The present inventors therefore sought to improve on the sensory experience and convenience of users, whilst maintaining skin permeation characteristics similar Voltaren Arthritis pain, Voltaren 1.16% Emulgel or Voltarol Joint Pain Relief 2.32% Gel. This approach was taken to maintain the advantageous properties of Voltaren 1%, 1.16% or 2.32% emulgel products, with which patients are familiar and appreciate. It was also taken in view of patient safety. By providing a product with a similar diclofenac permeation profile as marketed products with excellent safety characteristics, the risk of adverse events caused by the new product is reduced. Moreover, registration and approval of a new pharmaceutical product by regulatory authorities can be facilitated for products that are similar or comparable to already approved products. In one aspect, the inventors sought to develop a product which could be registered under the "well established use" criteria, relying on Voltaren 1% Emulgel or Voltaren 1.16% Emulgel or Voltarol Joint Pain Relief 2.32% Gel published literature data.

### COMPOSITIONS

### Hydroalcoholic single phase compositions

The compositions disclosed herein are compositions for topical drug delivery. This means, the compositions comprise an active pharmaceutical ingredient (API). The compositions are designed to be applied on skin of a patient who is in need of treatment with that API. The compositions are effective vehicles for the API to permeate skin. The compositions disclosed herein allow for the API to permeate through skin and to a site of action. The site of action may for example be a joint. Permeation of the API from a composition and through the skin can be determined in sophisticated pre-clinical experiments, such as the experiments descirbed later herein in the experimental section. Skin is a complex structure comprising several tissue layers. Therefore, the skin is a significant barrier for APIs. For the avoidance of doubt, the compositions of the present disclosure are not intended for treatment of a dermal condition. In one embodiment, the compositions are not for the treatment of a dermal condition.

The compositions are hydroalcoholic compositions. The term "hydroalcoholic composition" is intended to refer to a composition that is essentially free of oils, waxes and saturated carbohydrates, without precluding that the composition may comprise ingredients that comprise hydrophobic moieties.

The compositions described herein specifically are hydroalcoholic gels. "Hydroalcoholic gel" means a composition that comprises a gelled continuous phase comprising water and one or more alcohol(s). In other words, the compositions comprise a hydroalcoholic continuous phase. For the avoidance of doubt, hydroalcoholic gels can comprise additional ingredients besides water and alcohol(s).

The compositions are single phase compositions. "Single phase composition" means that the diclofenac or diclofenac salt is predominantly or completely dissolved in a solvent system and the solvents that constitute the solvent system are predominantly or completely miscible together. Single phase composition is meant to distinguish present compositions from emulsions, emulgels, colloidal mixtures, two phase compositions (e. g. oil and water), and the like. A present composition can be a single phase composition despite the mere presence of a gelling agent or an emulsifier. In this disclosure, gelling agent and gel network formed by gelling agent are not considered a separate or second phase. Moreover, micelles, formed by clustered emulsifier molecules, are not considered a separate or second phase.

Hydroalcoholic single phase gels are a different delivery format compared to conventional ointments. Ointments are single phase compositions comprising only a single, lipophilic phase. Ointments typically have a heavy consistency. Due to their lipophilicity, ointments have an occlusive effect on the skin, and can cause a greasy or tacky skin feel. Ointments also typically produce a warming effect. Yet different formats are emulsions, creams and emulgels. Emulsions are two phase compositions, also called two phase systems. These two phase systems comprise a lipophilic phase and a hydrophilic phase, formulated as a continuous phase and a dispersed phase. Emulsion compositions can either have a lipophilic continuous phase and a hydrophilic dispersed phase (so called water in oil emulsions, or W/O emulsions) or can have a hydrophilic continuous phase and a lipophilic dispersed phase (so called oil in water emulsions, or O/W emulsions). Emulsions comprise emulsifiers, i.e. molecules that have both, hydrophilic and lipophilic characteristics. Therefore, emulsifiers are amphiphilic molecules. Hydrophilic parts of emulsifier molecules are usually called head groups and lipophilic parts of emulsifier molecules are usually called tail groups. Emulsifiers help form and stabilise a dispersion of the lipophilic or hydrophilic phases. Without emulsifiers, emulsions would only form transiently, or would have poor stability, evidenced by un-mixing/ phase separation. This can occur because lipophilic and hydrophilic ingredients are usually immiscible, and the dispersed state is thermodynamically unstable. This un-mixing process can be referred to as phase-separation, because it leads to the separation of the dispersed phase and the continuous phase. Due to the amphiphilic nature of emulsifiers, they stabilise the inherently immiscible phases in a dispersed state. Creams and emulgels are a subgroup of emulsions. Creams are emulsions which can either have a lipophilic continuous phase and a hydrophilic dispersed phase (so called W/O creams) or can have a hydrophilic continuous phase and a lipophilic dispersed phase (so called O/W creams). Creams are also defined by their semi-solid consistency. Emulgels are emulsions with a gelled continuous phase. Emulgels can also have a lipophilic continuous phase and a hydrophilic dispersed phase (W/O) or can have a hydrophilic continuous phase and a lipophilic dispersed phase (O/W). However, commercial emulgel formats are usually O/W formats. Therefore, in the art, and in this disclosure, where there is referred to emulgels, typically it is referred to O/W emulgels.

The compositions disclosed herein are distinct from conventional multi-phase systems described above. The compositions disclosed herein are hydroalcoholic single phase gels. The compositions disclosed herein comprise a gelled hydroalcoholic continous phase. In that regard, the compositions described herein are similar to emulgels. However, emulgels additionally comprise a dispersed lipophilic phase. For example, Voltaren 1% Emulgel, Voltaren 1.16% Emulgel and Voltarol Joint Pain Relief 2.32% diclofenac DEA comprise a lipophilic dispersed phase, comprising, for example, liquid paraffin as a lipohpilic and hydrophobic ingredient. By contrast, the compositions of this disclosure do not comprise a lipophilic dispersed phase. In emulgels, the lipophilic phase enhances solubility of the active ingredient, in this case the diclofenac (salt). In single phase hydroalcohlic compositions, it is thus more challenging to solubilise diclofenac and diclofenac salts, especially at elevated concentrations of the API. Even if an amount of diclofenac (salt) is initially solubilised in a solvent or a composition, the API can have a strong tendency to re-crystallise. Therefore, it is challenging to formulate hydroalcoholic gels comprising diclofenac (salt) without a lipophilic dispersed phase, and to still achieve full solubilisation of the diclofenac (salt). It is especially challenging to formulate hydroalcoholic single phase gels wherein the diclofenac or diclofenac salt is fully solubilised over a prolonged period of time, over a shelf life, and/ or during storage.

The present inventors have identified hydroalcoholic single phase compositions that comprise a solvent system that can solubilise the diclofenac or diclofenac salts. The solvent system comprises a mixture of water, and particular concentrations of aliphatic C₂-C₄ mono-alcohol and of aliphatic diol. Unexpectedly, the inventors have found that by incorporation of particular concentrations of one or more non-ionic emulsifier(s) into the resulting hydroalcoholic single phase diclofenac gels, cumulative permeation of diclofenac can be reduced, such that the permeation characteristics can be tailored. Additionally, the resulting gels are optically transparent.

One aspect of the conventional multi-phase systems is that they typically comprise globules of a certain size. Most multi-phase systems comprise a dispersed phase with a globule size (also called particle size) of a size that scatters light and makes multi-phase systems appear cloudy or opaque, normally milky to opaque white. By contrast, the compositions described herein are clear, transparent, see-through, and/ or slightly opalescent. Without being bound to a specific theory, the present inventors suggest that the compositions disclosed herein comprise only structures with an average size too small to scatter light effectively. This results in transparent compositions. In these embodiments, the non-ionic emulsifier can be solubilised in the aqueous continuous phase. According to the theory of the inventors, in the disclosed compositions, the hydrophilic non-ionic emulsifier molecules can, from a certain concentration in the hydroalcoholic environment, associate to form small aggregates. The lipophilic tail groups of a number of non-ionic emulsifier molecules can associate to form micelles. The tail groups are located in cores of the micelles and the head groups face outwards, towards the hydrophilic continuous phase. Still, the compositions of this disclosure are not opaque, but are optically transparent. Moreover, the compositions are still considered single phase compositions as defined herein, even if micelles may be present.

In some embodiments of the compositions, structures formed by hydrophilic non-ionic emulsifier or micelles, if present, have an average globule size of below about 100nm, below about 90nm, below about 80nm, below about 70 nm, below about 60nm, below about 50nm or below about 40nm. In one particular embodiment of the compositions, structures formed by hydrophilic non-ionic emulsifier, or micelles, if present, have an average globule size of below about 50nm. In one embodiment, the compositions do not contain structures of an average globule size above 50nm, gelling agent excluded. Average globule size of structures of semi-solid compositions can be measured with a compound microscope with or without particle count software.

In one embodiment, the compositions are not coloured. In one embodiment, the compositions are colourless. In one embodiment, the compositions disclosed herein are clear to opalescent. In one embodiment, the compositions are clear. In one embodiment, the compositions do not significantly scatter light in the visible to human spectrum. In one embodiment, the compositions do not significantly scatter light in the range of about 380nm to about 780nm wavelength. In one embodiment, the compositions are see-through. In one embodiment, the compositions are transparent. In one embodiment, the compositions do not contain a dispersed phase which scatters light in the visible to human spectrum, and the compositions are transparent to opalescent. In one embodiment, the compositions of this disclosure are hydroalcoholic single phase gels, only comprising structures that don't scatter light in the visible to human spectrum. In particular embodiments of the embodiments described above, micelles, if present, have an average globule size of below about 100nm, below about 90nm, below about 80nm, below about 70 nm, below about 60nm, below about 50nm or below about 40nm.

Advantageously, the optical attributes of the compositions described herein have a modern and attractive look that appeals to many patients and consumers. Such a "clear like water" appearance can be a visible marker that the compositions are primarily composed of water, which can also be perceived positively. Moreover, the compositions are easier to dose with dosing cards for topical products such as the dosing card in Fig. 1. When dosing the compositions of the invention on a dosing card, a user can perceive dose indicators on the dosing card, even when they have already expelled the composition onto the dosing card. This enhances dosing security and dosing accuracy. This also improves the patients' dosing skills.

*"Substantially free or"*, in the present disclosure, is to be understood as meaning that no additional ingredients are added to the formulation that have a certain functionality. For example, *"substantially free of permeation enhancers"*, means that no permeation enhancer is added to the compositions. This however does not exclude the ingredients explicitly listed in this disclosure as mandatory or optional or preferred components. The skilled person understands that, in pharmaceutical compositions, many ingredients exert more than one function. It thus may be that an ingredient which listed as a mandatory or optional or preferred component herein, in addition to its functionality described herein, exerts certain other functionalities. For example, a given alcohol that is described as one component of the compositions, may act as a solubiliser, but can in addition also influence permeation, for example it may have a permeation enhancing effect in some concentrations. In this example, *"substantially free of permeation enhancers"* would not exclude the presence of this solvent in the compositions of this disclosure. Additionally, compositions may contain insignificant or trace amounts of certain compounds, e.g. due to impurities, or due to degradation of ingredients. This is also not encompassed and such compositions would still be considered to be *"substantially free of"* that compound. "Substantially free from lipophilic excipients" does not preclude presence of hydrophilic non-ionic emulsifier in the compositions. The hydrophilic non-ionic emulsifiers used in the present compositions are not considered lipophilic excipients, although a *part* of their molecule structure, the tail group, is lipophilic. Hydrophilic non-ionic emulsifiers are amphiphilic molecules that comprise lipophilic and hydrophilic moieties, wherein in total, the hydrophilic characteristics predominate and render the emulsifier hydrophilic and water-soluble.

Lipophilicity of a compound can be assessed for example with the shake-flask method and can be expressed in a logP value. The logP value (partition coefficient or distribution coefficient) is an indicator how a given molecule partitions between a lipophilic organic phase (typically n-octanol) and a polar aqueous phase (typically de-ionised water). In some embodiments, the compositions are substantially free from excipients with a logP_{octanol/water} value of above about 3, above about 4, above about 5 or above about 6 (except for diclofenac or its salts). Preferably, the compositions do not contain a lipophilic or fatty phase. This is preferred to provide the consumers a "fat-free", "no-messy", "non-greasy" alternative, based on water and alcohols. The compositions leave no lipophilic traces on the application site, the hands or on clothes. Moreover, the compositions are transparent.

Although the compositions are free from a lipophilic phase, they comprise a hydrophilic non-ionic emulsifier. As explained above, in conventional multi-phase systems, emulsifiers are used to achieve a dispersion of one immiscible phase in another. In compositions without a lipophilic phase, no emulsifiers are generally incorporated, as there is no dispersed phase that needs to be stabilised in the aqueous phase, and there is no risk of phase separation. Therefore, typically, hydroalcoholic compositions of the art do not contain any emulsifiers. Contrary to this art, the compositions of this disclosure comprise hydrophilic non-ionic emulsifiers, despite them being hydroalcoholic compositions without a lipophilic phase. Emulsifiers have also been reported to have permeation enhancing effects in multi-phase systems (see for example Iti Som, Kashish Bhatia, and Mohd. Yasir, "Status of surfactants as penetration enhancers in transdermal drug delivery", J Pharm Bioallied Sci., 2012 Jan-Mar; 4(1): 2-9;doi: 10.4103/0975-7406.92724). Contrary to these teachings, in the compositions of this disclosure, the hydrophilic non-ionic emulsifiers have a permeation reducing effect.

The present inventors have surprisingly found that in the hydroalcoholic compositions of this disclosure, non-ionic emulsifiers can reduce cumulative permeation of diclofenac. In one aspect, non-ionic emulsifiers thus can have a *permeation retarding* effect on diclofenac or its salts in the hydroalcoholic single phase compositions of this disclosure. This is particularly surprising in view of the teachings in the art (Iti Som et al, referenced above) about *permeation enhancing effects* of non-ionic emulsifiers. Moreover, the present inventors found that particular combinations of non-ionic emulsifiers are especially suitable to reduce cumulative permeation of diclofenac.

As will be evident from the example section, the present inventors found that diclofenac or its salts show a higher permeation rate from hydroalcoholic compositions, compared to emulgel compositions (see, for example, Example 1) comprising the same concentration of API and other excipients. As discussed above, the inventors sought to provide compositions that were as safeas and and for which it would be possible to rely on the vast drug safety experience of the established product Voltarol Joint Pain Relief 2.32% diclofenac DEA. Therefore, in one aspect, the inventors aimed at providing compositions that have a permeation profile similar to Voltarol Joint Pain Relief 2.32% diclofenac DEA. Surprisingly, it was found that cumulative permeation of diclofenac from hydroalcoholic gels can be tailored by incorporating hydrophilic one or more non-ionic emulsifier(s). Furthermore, non-ionic emulsifiers do not contain an ionisable group and are relatively insensitive to changes in pH. Therefore, non-ionic emulsifiers can stabilise the compositions.

In one embodiment, the compositions comprise at least one non-ionic emulsifier. In one embodiment the compositions comprise at least two different classes of non-ionic emulsifier. In one embodiment, the compositions comprise one or more non-ionic emulsifiers. In one embodiment, the compositions comprise at least two different non-ionic emulsifiers. In yet another embodiment, the compositions comprise two non-ionic emulsifiers and no further emulsifiers. In some embodiments, the compositions comprise at least two non-ionic emulsifiers. In another embodiment, the compositions comprise exactly two non-ionic emsulifiers.

In one embodiment, the compositions comprise a non-ionic emulsifier with a hydrophilic-lipophilic balance value of about 13 to about 17, about 14 to about 16, or about 15. In one particular embodiment, the compositions comprise a non-ionic emulsifier with a hydrophilic-lipophilic balance value of about 15. In one embodiment, the compositions comprise one non-ionic emulsifier, wherein the one non-ionic emulsifier has a hydrophilic-lipophilic balance value of about 13 to about 17, about 14 to about 16, or about 15. In any of these embodiments, the described non-ionic emulsifier can be present in a concentration of about 0.1% w/w to about 5% w/w, about 1% w/w to about 3% w/w, about 1.5% w/w to about 2.5% w/w or about 2% w/w.

In some embodiments, the compositions comprise a non-ionic emulsifier with a hydrophilic-lipophilic balance value of about 15 to about 19, about 16 to about 18, or about 17. In one particular embodiment, the compositions comprise a non-ionic emulsifier with a hydrophilic-lipophilic balance value of about 17. In one embodiment, the compositions comprise one non-ionic emulsifier, wherein the one non-ionic emulsifier has a hydrophilic-lipophilic balance value of about 15 to about 19, about 16 to about 18, or about 17. In any of these embodiments, the described non-ionic emulsifier can be present in a concentration of about 0.1% w/w to about 5% w/w, about 0.5% w/w to about 1.5% w/w, about 0.7% w/w to about 1.3% w/w or about 1% w/w.

In another embodiment, the compositions comprise a first non-ionic emulsifier and a second non-ionic emulsifier, wherein the first non-ionic emulsifier is different from the second non-ionic emulsifier. The first and the second non-ionic emulsifier may have the same lipophilic balance value or different lipophilic balance values. In one embodiment, the compositions comprise a first non-ionic emulsifier with a hydrophilic-lipophilic balance value of about 14 to about 16, and a second non-ionic emulsifier with a hydrophilic-lipophilic balance value of about 16 to about 18. In yet another embodiment, the compositions comprise at least two non-ionic emulsifiers, wherein one of the at least two non-ionic emulsifiers has a hydrophilic-lipophilic balance value of about 13 to about 17, about 14 to about 16, or about 15. In one particular embodiment, the compositions comprise two non-ionic emulsifiers, wherein one of the two non-ionic emulsifiers has a hydrophilic-lipophilic balance value of about 13 to about 17, about 14 to about 16, or about 15.

In another embodiment, the compositions comprise at least two non-ionic emulsifiers, wherein one of the at least two non-ionic emulsifiers has a hydrophilic-lipophilic balance value of about 15 to about 19, about 16 to about 18, or about 17. In one particular embodiment, the compositions comprise two different non-ionic emulsifiers, wherein one of the two non-ionic emulsifiers has a hydrophilic-lipophilic balance value of about 15 to about 19, about 16 to about 18, or about 17.

In one particular embodiment, the compositions comprise a first non-ionic emulsifier with a hydrophilic-lipophilic balance value of about 13 to about 17, about 14 to about 16, or about 15, and a second non-ionic emulsifier with a hydrophilic-lipophilic balance value of about 15 to about 19, about 16 to about 18, or about 17. The first non-ionic emulsifier can be present in a concentration of about 0.1% w/w to about 5% w/w, 1% w/w to about 3% w/w, about 1.5% w/w to about 2.5% w/w or about 2% w/w. The second non-ionic emulsifier can be present in a concentration of about about 0.1% w/w to about 5% w/w, 0.5% w/w to about 1.5% w/w, about 0.7% w/w to about 1.3% w/w or about 1% w/w. In another particular embodiment, the compositions comprise one non-ionic emulsifier with a hydrophilic-lipophilic balance value of about 15 and one non-ionic emulsifier with a hydrophilic-lipophilic balance value of about 17. The non-ionic emulsifier with a hydrophilic-lipophilic balance value of about 15 can be present in a concentration of about 0.1% w/w to about 5% w/w, 1% w/w to about 3% w/w, about 1.5% w/w to about 2.5% w/w or about 2% w/w. The non-ionic emulsifier with a hydrophilic-lipophilic balance value of about 17 can be present in a concentration of about 0.1% w/w to about 5% w/w, 0.5% w/w to about 1.5% w/w, about 0.7% w/w to about 1.3% w/w or about 1% w/w.

In one embodiment, the compositions comprise a hydrophilic non-ionic emulsifier which is an ethoxylated C₁₆ to C₁₈ alcohol. In one embodiment, the compositions comprise a non-ionic emulsifier selected from the group consisting of Macrogol Cetostearyl Ether 20, Polysorbate 60, Polysorbate 80, Isosteareth 20, PEG-60 Almond Glycerides, PEG-20 Methyl Glucose Sesquistearate, Oleth-20, Steareth-20, and combinations of two or more thereof. In one particular embodiment, the compositions comprise macrogol Cetostearyl ether. In another particular embodiment, the compositions comprise macrogol cetostearylether 20. Macrogol cetostearyl ether 20 is a compendial name used by the European Pharmacopoeia (Ph.Eur.). In the United States Pharmacopoeia (USP), the name Polyoxyl 20 cetostearyl ether (USP) is used for the same ingredient. In the International Nomenclature of Cosmetic Ingredients (INCI), the name Ceteareth-20 (INCI) is used for the same ingredient. One trade name for macrogol cetostearyl ether 20 is Kolliphor CS 20. In any of these embodiments, the defined non-ionic emulsifier can be present in a concentration of about 0.1% w/w to about 5% w/w, 1% w/w to about 3% w/w, about 1.5% w/w to about 2.5% w/w or about 2% w/w.

In one embodiment, the compositions comprise a non-ionic emulsifier comprising a 2-isopropyl-tetrahydrofuran moiety comprising one or more oligo(ethylene oxide) ester group, wherein all ethylene oxide units of the oligo(ethylene oxide) ester groups in combination comprise about 10 to about 30, about 14 to about 24, or about 20 carbon atoms. In one particular embodiment, the oligo(ethylene oxide) ester groups comprise a lauric acid ester. In another embodiment, the compositions comprise a polysorbate. In another embodiment, the compositions comprise a non-ionic emulsifier selected from the group consisting of Polysorbate 20, Laureth-20, and combinations thereof. In one particular embodiment, the compositions comprise Polysorbate 20. Polysorbate 20 is a compendial name used in the Ph.Eur. and the USP. One trade name for Polysrobate 20 is Kolliphor PS 20. In any of these embodiments, the defined non-ionic emulsifier can be present in a concentration of about 0.1% w/w to about 5% w/w, 0.5% w/w to about 1.5% w/w, about 0.7% w/w to about 1.3% w/w or about 1% w/w.

In one embodiment, the compositions comprise a first non-ionic emulsifier which is a macrogol cetostearyl ether and a second non-ionic emulsifier which is a polysorbate. The macrogol cetostearyl ether can be present in a concentration of about 0.1% w/w to about 5% w/w, about 1% w/w to about 3% w/w, about 1.5% w/w to about 2.5% w/w or about 2% w/w. The polysorbate can be present in a concentration of about 0.1% w/w to about 5% w/w, 0.5% w/w to about 1.5% w/w, about 0.7% w/w to about 1.3% w/w or about 1% w/w. In yet another particular embodiment, the compositions comprise a combination of Cetostearylether 20 and Polysorbate 20. The Cetostearylether 20 can be present in a concentration of about 0.1% w/w to about 5% w/w, about 1% w/w to about 3% w/w, about 1.5% w/w to about 2.5% w/w or about 2% w/w. The Polysorbate 20 can be present in a concentration of about 0.1% w/w to about 5% w/w, about 0.5% w/w to about 1.5% w/w, about 0.7% w/w to about 1.3% w/w or about 1% w/w.

In particular embodiments of any of the embodiments described above, the listed emulsifier(s) is/are the sole emulsifier(s) in the composition. In some embodiments, the compositions are substantially free of anionic emulsifier. In some embodiments, the above compositions are substantially free of anionic emulsifier and substantially free of cationic emulsifier.

In one embodiment, the compositions comprise non-ionic emulsifier in a total concentration of about 0.1% w/w to about 5% w/w, 0.5% w/w to about 4% w/w, about 1% w/w to about 3.5% w/w, or about 1.5% w/w to about 3% w/w. In one embodiment, the compositions comprise non-ionic emulsifier in a total concentration about 2% w/w . In another embodiment, the compositions comprise non-ionic emulsifier in a total concentration about 3% w/w.

### Solvent system

The compositions disclosed herein comprise a solvent system that comprises water, aliphatic C₂-C₄ mono-alcohol and aliphatic diol. In one embodiment, the compositions described herein comprise more than about 50% w/w, more than about 60% w/w or more than about 70% w/w water. In one particular embodiment, the compositions comprise about 65% w/w to about 75% w/w water. In one embodiment, the compositions are water-based. In one embodiment, the compositions are aqueous compositions. Advantageously, water imparts a cooling effect on the skin. It is also a readily available ingredient with a low environmental impact. Furthermore, water is a clear ingredient, which is advantageous in view of the importance of the optic appearance of the final compositions as described above. The water can be purified water, distilled water, or similar. The water can be pharma grade water. In one embodiment, the compositions comprise water as a composition base. Being part of the solvent system, water also plays a role in solubilising diclofenac or diclofenac salts.

In addition to water, the solvent system comprises aliphatic C₂-C₄ mono-alcohol. The compositions can comprise a single aliphatic C₂-C₄ mono-alcohol or multiple aliphatic C₂-C₄ mono-alcohols. In one embodiment, the aliphatic C₂-C₄ mono-alcohol is selected from ethanol, propanol, isopropanol, butan-1-ol, and a combination of two or more thereof. In some embodiments, the compositions comprise isopropanol. In other embodiments, the compositions comprise ethanol. In one embodiment, the compositions comprise ethanol, isopropanol, or both. In one embodiment, the compositions comprise water, ethanol and isopropanol. In one embodiment, isopropanol is used as the sole aliphatic C₂-C₄ mono-alcohol. In any of the above embodiments, the water and the alcohol(s) are present in a continuous phase of the compositions. In some embodiments, the compositions comprise a continuous phase comprising water, isopropanol and optionally, ethanol. In one particular embodiment, the compositions comprise a continuous phase comprising water and isopropanol.

Compared to water alone, C₂-C₄ mono-alcohol(s) provide a better solubilisation of diclofenac and diclofenac salts. This facilitates diclofenac solubilisation and reduces the risk of re-crystallisation. Moreover, aliphatic C₂-C₄ mono-alcohols yield topical gels with a reduced drying time compared to pure water based gels. Upon application of a compositions to the skin, aliphatic C₂-C₄-mono-alcohol(s) can evaporate. Evaporation after application on skin reduces drying time. Drying time can be described as the time that elapses from application of a topical product onto skin and up until the skin on the application site feels dry again. Moreover, aliphatic C₂-C₄-mono-alcohols, can impart a pronounced cooling effect to topical compositions. After application of a topical composition onto skin, evaporation of composition components is driven by thermal energy from the application site. The evaporation process therefore draws thermal energy from the application site. Thereby, a cooling sensation on the skin is produced. This cooling effect is beneficial for patients and consumers: "Calor", i.e. heating of the affected body area, is one of the cardinal symptoms of inflammatory processes. Cooling of the administration site can therefore sooth inflammatory processes, and can ease inflammation pain. Moreover, cooling can produce a perception of immediate action of the topical product. Additionally, aliphatic C₂-C₄-mono-alcohols are transparent. They are therefore suitable for the formulation of a topical composition with the desired optical transparency.

In some particular embodiments, the compositions comprise isopropanol as the sole aliphatic C₂-C₄ mono-alcohol. In some embodiments, the compositions comprise a continuous phase comprising water and isopropanol, and are free of ethanol. In some embodiments, the compositions comprise a continuous phase comprising water and isopropanol, and do not comprise any other C₂-C₄-mono-alcohols. Advantageously, by using isopropanol as the sole aliphatic C₂-C₄ mono-alcohol, the number of different In these embodiments, no additional aliphatic C₂-C₄ mono-alcohol is required to solubilise the diclofenac or the diclofenac salts, and to provide a cooling sensation on the skin. Especially, it is not required to add another aliphatic C₂-C₄ mono-alcohol that evaporates more readily, such as ethanol. This can be preferred in some embodiments, because ethanol is regulated in many states for tax or moral reasons. Therefore, in some embodiments, the compositions do not comprise ethanol.

Aliphatic C₂-C₄ mono-alcohol is useful to reduce drying time, which can generally be beneficial. However, as alcohol component(s) of the compositions also play a role in API solubilisation, the solvent system may not evaporate too readily, and adequate concentrations of the API need to be maintained in solubilisation. Thereby, risk of crystallisation or precipitation of API after application on the skin is reduced. Thereby, the amount of API which is bioavailable can be enhanced. This is advantageous for efficacy, patient safety and environmental safety. It is also preferred as, thereby, residue formation on the skin after application and drying can be reduced. This provides for a pleasant patient experience. Therefore, the solvent system used in the present compositions further comprises an aliphatic diol as a co-solvent.

The aliphatic diol can in some embodiments be a C₂-C₄ polyalcohol. In some embodiments, the compositions disclosed herein comprise a glycol. In one embodiment, the compositions comprise an aliphatic diol selected from the group of ethylene glycol, propylene glycol and 1,3-butylene glycol, and any combinations thereof. In some embodiments, propylene glycol is present as a sole aliphatic diol.

The aliphatic diol or C₂-C₄ polyalcohol can act as a co-solubiliser of the API. In contrast to the aliphatic C₂-C₄ mono-alcohol(s) discussed above, aliphatic diol or C₂-C₄ polyalcohol doesn't evaporate readily after application onto skin. The aliphatic diol or C₂-C₄ polyalcohol therefore remains in the topical composition on the skin, whilst aliphatic C₂-C₄ mono-alcohol(s) evaporate. Thereby, the aliphatic diol or C₂-C₄ polyalcohol can maintain the API in solubilised state after application onto the skin.

Moreover, aliphatic diols or C₂-C₄ polyalcohols are hygroscopic. They can impart humectant, water retaining properties to topical compositions. This can provide a pleasant skin feeling, such as a feeling of soft, supple, or nurtured skin. This can also help to balance lipid-extracting properties of C₂-C₄ mono-alcohol(s). These effects are of particular importance in single phase hydroalcoholic compositions, as these do not comprise a lipophilic phase that could provide emollient or fat-restoring properties.

The aliphatic diols or C₂-C₄ polyalcohols can also influence permeation properties of a formulation. However, this effect on permeation is also dependent on their concentration and on other components and the overall composition. As discussed above, the present inventors sought to formulate a composition with permeation characteristics similar to those of the existing Voltaren 1% or 1.16% or 2.32% Emulgel products, which is explained in more detail in a separate section below.

The aliphatic C₂-C₄ mono-alcohol(s) can be present in the compositions in a concentration of about 5% w/w to about 20% w/w. Preferably, the aliphatic C₂-C₄ mono-alcohol (s) can be present in a concentration of about 8% w/w to about 15% w/w. In one embodiment, the aliphatic C₂-C₄ mono-alcohol (s) is present in a concentration of about 12.5% w/w. In particular embodiments of any of the above embodiments, the aliphatic C₂-C₄ mono-alcohol (s) can be isopropanol.

In particular embodiments, the compositions comprise isopropanol in a concentration of about 5% w/w to about 20% w/w, about 8% w/w to about 15% w/w or about 12.5% w/w. In one embodiment, the compositions of the present disclosure comprise isopropanol in a concentration of about 5% w/w to about 20% w/w, about 8% w/w to about 15% w/w or about 12.5% w/w, and do not comprise any other aliphatic C₂-C₄ mono-alcohol.

Aliphatic C₂-C₄-mono-alcohol(s) in these concentration ranges strike a balance. On the one hand, high concentrations of C₂-C₄-mono-alcohol(s) might be beneficial for API solubilisation, microbial preservation and/ or cooling effects. On the other hand, high concentrations of aliphatic C₂-C₄-mono-alcohol(s) can result in a final product which is classified as hazardous material in some regulations, or may be subject to additional regulatory scrutiny, potentially complicating production, supply and regulatory logistics. At high aliphatic C₂-C₄ mono-alcohol concentrations, compositions may require particular tight packaging to avoid evaporation of the aliphatic C₂-C₄ mono-alcohol during storage. Compositions comprising aliphatic C₂-C₄-mono-alcohol(s) in the concentration ranges described above don't require to be labelled, stored, and handled under high scrutiny, as they are not classified as hazardous material, and do not require particular tight packaging. This is desirable, because such a classification can complicate manufacture and supply, and may deter consumers from using the final product. Moreover, with too high concentrations of aliphatic C₂-C₄-mono-alcohol(s), API precipitation on the application site, as described above, may occur. The described concentration ranges for aliphatic C₂-C₄-mono-alcohol(s) can, in combination with the water and the aliphatic diol of the solvent system, sufficiently solubilise the API. The compositions thus have reduced risk of leaving API residue or precipitate on skin.

Additionally, described concentrations can produce an adequate cooling sensation upon application. The initial onset of a cooling sensation can distract patients from the inflamed area of pain and produce a quick feeling of onset of pain relief. This physiologically helps patients with quick pain relief. In particular, the cooling sensation can be perceived as pronounced or strong, with a quick onset and can be perceived as long lasting.

Aliphatic mono-alcohols additionally have anti-microbial properties. If included in sufficiently high concentrations in the compositions, the compositions have reduced risk of microbiotic contamination and bacterial growth. This is particularly important for the compositions comprising high concentrations of water, such as more than about 50% w/w, more than about 60% w/w, or more than about 65% w/w water.

In another aspect, aliphatic C₂-C₄-mono-alcohol(s) can have an odour that may be deterring some consumers. Perfume or other odour masking agents can be required to mask the alcoholic odours. However, incorporation of perfume can in some examples be challenging or not desired. Surprisingly, compositions comprising aliphatic C₂-C₄-mono-alcohol(s) within the described concentration ranges can have a pleasant odour. Therefore, they are particularly suitable to be formulated as perfume-free compositions. In one particular embodiment, the compositions are perfume-free.

In yet another aspect, the concentration ranges described above also provide for skin-friendly compositions. Aliphatic C₂-C₄-mono-alcohol(s) have some lipid-extracting (lipolytic) properties. These lipid-extracting properties can affect the natural fat and ceramide components and barrier function of skin. It can have dehydrating, degreasing and irritating effects to the skin. In some cases, this can lead to dry skin, flaky skin, and/ or tight feeling on the skin. On the other hand, aliphatic C₂-C₄-mono-alcohol(s) can have a permeation enhancing effect for the API. In conventional topical compositions that comprise a lipophilic phase, the lipophilic components can balance dehydrating, degreasing and irritating effects of alcohol components. As discussed above, the compositions of this disclosure do not comprise a lipophilic dispersed phase. Still, the compositions of this disclosure, due to their balanced blend of ingredients, are very skin friendly, as they comprise a suitable concentration of aliphatic C₂-C₄ mono-alcohol, and further comprise a co-solvent which is an aliphatic diol.

The co-solvent, an aliphatic diol or C₂-C₄-polyalcohol can be present in the compositions in an amount of about 2.5% w/wto about 20% w/w. Preferably, aliphatic diol or C₂-C₄-polyalcohol can be present in the compositions in an amount of about 3% w/w to about 15% w/w. In one embodiment, aliphatic diol or C₂-C₄-polyalcohol is present in an amount of about 6% w/w to about 12% w/w. In yet another embodiment, aliphatic diol or C₂-C₄-polyalcohol is present in an amount of about 8% w/w to about 10% w/w. In one embodiment, the compositions comprise about 8% w/w of aliphatic diol. In another embodiment, the compositions comprise about 10% w/w of aliphatic diol.

Benefits of including a certain concentration of aliphatic diol or C₂-C₄-polyalcohol have been described above. However, the present investors found that the described particular concentration ranges of aliphatic diol or C₂-C₄-polyalcohol are particularly useful. Aliphatic diol or C₂-C₄-polyalcohol in the described concentration ranges yield compositions with a reduced risk of skin irritation. In another aspect, the described ranges yield compositions with an acceptable drying time. In again another aspect, the resulting compositions have very pleasant skin application attributes. For example, they are not perceived as sticky or greasy. Moreover, the resulting compositions are clear, transparent, or see-through. In some embodiments, the compositions can be opalescent. Moreover, aliphatic diol or C₂-C₄-mono-alcohol also has an influence on permeation characteristics of a composition.

In one embodiment, the compositions comprise an aliphatic C₂-C₄ mono-alcohol which is isopropanol and an aliphatic diol which is propylene glycol.

The total combined amount of aliphatic C₂-C₄-mon-alcohol and aliphatic diol or C₂-C₄-polyalcohol preferably is more than about 10% w/w. The total combined amount of aliphatic C₂-C₄-mono-alcohol and aliphaticdiol or C₂-C₄-polyalcohol preferably is less than about 30% w/w. Expressed in ranges, the total combined amount of aliphatic C₂-C₄-mono-alcohol and aliphatic diol or C₂-C₄-polyalcohol can be about 15% w/w to about 25% w/w, preferably about 19% w/w to about 24% w/w. In one embodiment the total combined amount of aliphatic C₂-C₄-mono-alcohol and aliphatic diol C₂-C₄-polyalcohol is about 20.5% w/w. In one embodiment the total combined amount of aliphatic C₂-C₄-mono-alcohol and aliphatic diol or C₂-C₄-polyalcohol is about 22.5% w/w.

In particular embodiments, the aliphatic C₂-C₄ mono-alcohol is isopropanol and the aliphatic diol is propylene glycol. In some of these embodiments, the total combined concentration of isopropanol and propylene glycol is more than about 10% w/w. The total combined concentration of isopropanol and propylene glycol preferably is less than about 30% w/w. Expressed in ranges, the total combined concentration of isopropanol and propylene glycol can be about 10% w/w to about 30% w/w, preferably about 12% w/w to about 25% w/w. In particular embodiments, the total combined concentration of isopropanol and propylene glycol is about 14% w/w, about 15% w/w, about 15.5% w/w, about 17.5% w/w, about 20% w/w or about 25% w/w.

In some embodiments, the ratio of aliphatic diol to aliphatic C₂-C₄ mono-alcohol is 1:0.5 to 1:2. In some embodiments, ratio of aliphatic diol to aliphatic C₂-C₄ mono-alcohol is 1:0.5 to 1:2, 1:1.9, 1:1.8,1:1.17,1:1.16,1:1.15,1:1.14,1:1.13,1:1.125,1:1.12, 1:1.11, 1:1, 1:0.9, 1:0.8, 1:0.7 or 1:0.6. In particular embodiments, the ratio of aliphatic diol to aliphatic C₂-C₄ mono-alcohol is 1:1.4 to 1:1. In one particular embodiment, the ratio of aliphatic diol to aliphatic C₂-C₄ mono-alcohol is 1:1.25. In particular embodiments of the above embodiments, the aliphatic C₂-C₄ mono-alcohol is isopropanol, and the aliphatic diol is propylene glycol.

In other particular embodiments, the compositions comprise about 10% w/w, about 11% w/w or about 12.5% w/w isopropanol and about 5% w/w or about 10% w/w propylene glycol. In a particular embodiment, the compositions comprise about 12.5% w/w isopropanol and about 10% w/w propylene glycol.

The compositions disclosed herein comprise an API which is diclofenac, or a pharmaceutically acceptable salt of diclofenac. This includes deuterated forms of diclofenac or its salts, in which one or more hydrogen atoms in the diclofenac molecule is/are replaced with a deuterium atom (i.e. deuterated diclofenac). In one embodiment, the compositions comprise diclofenac, diclofenac ammonium, diclofenac sodium or diclofenac potassium. In one embodiment, the compositions comprise a pharmaceutically acceptable inorganic diclofenac salt. In one particular embodiment, the compositions comprise diclofenac ammonium, diclofenac sodium or diclofenac potassium. In another particular embodiment, the compositions comprise diclofenac sodium or diclofenac potassium. In yet another embodiment, the compositions comprise diclofenac sodium. In some embodiments, the compositions comprise diclofenac or a pharmaceutically acceptable salt of diclofenac, as the sole active pharmaceutical principle.

The present inventors compared the stability of conventional emulgels and hydroalcoholic gel formulations, both comprising diclofenac diethylammonium. They found that emulgels showed a slower nitrosamine formation than hydroalcoholic compositions. Without being bound to a specific theory, the present inventors believe that a lipophilic phase, and antioxidant that may be present in a lipophilic phase, can have a stabilising effect on secondary amines, and thereby slow down nitrosamine formation. Therefore, in the hydroalcoholic single phase compositions of this disclosure, inorganic diclofenac salts, for example diclofenac ammonium or diclofenac sodium diclofenac potassium can be preferred. However, these salts can be more challenging to solubilise in hydroalcoholic gels than organic diclofenac salts. Moreover, the counter ions, especially Na⁺ and K⁺, can interact with certain gelling agents and negatively affect their gel building and viscosifying effect. This is, for example, the case with so-called Carbomer gelling agents, which are often referred to as "ion-sensitive". However, Carbomer gelling agents are preferred gelling agents for the compositions of this disclosure, because they produce gels with a particularly appealing haptics and skin feel. The present inventors have surprisingly been able to formulate embodiments of the presently disclosed compositions that comprise inorganic diclofenac salts, and a Carbomer gelling agent, in a hydroalcoholic single phase formulation base, that still have a suitable viscosity.

Preferably, the compositions are substantially free of additional API(s). This means, that no API in addition to diclofenac (or diclofenac salt) is added to the compositions. For example, the compositions do not contain an additional NSAID, such as ibuprofen. In one embodiment, the compositions do not comprise a counter-irritant. In one embodiment, the compositions are substantially free of a counter-irritant.

In some embodiments, the compositions comprise diclofenac or a pharmaceutically acceptable salt of diclofenac in a total concentration of about 1.5% w/w to about 2.5% w/w of diclofenac sodium equivalent. Generally, in this disclosure, concentrations of diclofenac salts are given as diclofenac sodium equivalents, if not specified otherwise. In some embodiments, the compositions comprise diclofenac or a pharmaceutically acceptable salt of diclofenac in a total concentration about 1.8% w/w to about 2.2% w/w of diclofenac sodium equivalent. In some embodiments, the compositions comprise about 2.32% w/w diclofenac diethylammonium salt. In some embodiments, the compositions comprise about 1.5% w/w, about 1.6% w/w, about 1.7% w/w, about 1.8% w/w, about 1.9% w/w, about 2.0% w/w, about 2,1% w/w, about 2.2% w/w, about 2.3% w/w, about 2.4% w/w, or about 2.5% w/w diclofenac sodium. In a particular embodiment, the compositions comprise about 2% diclofenac sodium. As discussed above, higher concentrations of diclofenac or its salts are more challenging to solubilise. Moreover, concentrations of counter-ions can disturb gelling capacity of the gelling agent. The disclosed solvent systems are suitable to solubilise even the recited high concentrations of diclofenac or diclofenac salts, and the resulting hydroalcoholic single phase gel compositions are optically transparent. They still have the desired spread characteristics and viscosity, even if a Carbomer gelling agent is used. Moreover, they have the desired permeation characteristics.

In alternative embodiments, the compositions of this disclosure can comprise diclofenac or a diclofenac salt in a concentration of about 0.5% w/w to about 1.5% w/w diclofenac sodium equivalent. In some embodiments, the compositions comprise diclofenac or a pharmaceutically acceptable salt of diclofenac in a total concentration of about 0.8% w/w to about 1.2% w/w of diclofenac sodium equivalent. In one embodiment, the compositions comprise diclofenac sodium in a concentration of about 0.5% w/w to about 1.5% w/w. Preferably, the compositions comprise diclofenac sodium in a concentration of about 1% w/w to about 1.2% w/w. Especially preferred is diclofenac sodium in a concentration of about 1% w/w. In alternative embodiments, the compositions comprise about 1.16% w/w diclofenac diethylammonium salt. In some other embodiments, the compositions comprise about 0.5% w/w, about 0.6% w/w, about 0.7% w/w, about 0.9% w/w, about 1.0% w/w, about 1.1% w/w, about 1.2% w/w, about 1.3%w/w, about 1.4% w/w, or about 1.5% w/w diclofenac sodium.

Within these recited concentration ranges, excellent API solubilisation is achieved. No visible crystals of API remain in the compositions after formulation. In one embodiment, no crystals of diclofenac or its salts are detectable in the compositions upon inspection with the naked eye, or upon inspection with a light microscope with a ×10, ×40, ×100, ×400, ×800 or ×1200 magnification.

Preferably, the compositions are substantially free of additional API(s). This means, that no API in addition to diclofenac (salt) is added to the compositions. For example, the compositions do not contain an additional NSAID, such as ibuprofen. In one embodiment, the compositions do not comprise a counter-irritant. In one embodiment, the compositions are substantially free of a counter-irritant. In one embodiment, the compositions comprise diclofenac or a pharmaceutically acceptable salt of diclofenac as the sole active pharmaceutical principle.

The compositions of this disclosure can comprise diclofenac or a diclofenac salt in a concentration of about 1.5% w/w to about 2.5% w/w, 1.8% w/w to about 2.2% w/w, or about 1.9% w/w to about 2.1% w/w diclofenac sodium equivalent. Generally, in this disclosure, concentrations of diclofenac salts are given as diclofenac sodium equivalents, if not specified otherwise. In one embodiment, the compositions comprise diclofenac sodium in a concentration of about 1.5% w/w to about 2.5% w/w. Preferably, the compositions comprise diclofenac sodium in a concentration of about 1.8% to about 2.2% w/w. Especially preferred is diclofenac sodium in a concentration of about 2% w/w.

Within these concentration ranges, excellent API solubilisation is achieved. No visible crystals of API remain in the compositions after formulation. In one embodiment, no crystals of diclofenac or its salts are detectable in the compositions upon inspection with the bear eye, or upon inspection with a light microscope with a ×10, ×40, ×100, ×400, ×800 or ×1200 magification.

The compositions disclosed herein comprise a gelling agent. The compositions comprise an hydroalcoholic continous phase comprising a gelling agent. In one embodiment the compositions comprise a gelled hydroalcoholic continous phase. In one embodiment, the compsitions are gels comprising an aqueous continous phase and a three-dimensional network formed by a gelling agent.

In some embodiments, the compositions of the disclosure can comprise a Carbomer gelling agent. Carbomer gelling agents yield very clear, transparent gels. As mentioned above, Carbomer gelling agents also yield gels with superior spreading characteristics that are preferred by many consumers over other gels, such as hydroxypropyl methyl cellulose gels. For example, Carbomer 974P or Carbomer 980 can be used.

The Carbomer gelling agent can be present in amounts sufficient to raise the viscosity of the compositions. The carbomer gelling agent can be present in concentrations of about 1.5% w/w to about 2.5% w/w, about 1.8%w/w to about 2.2% w/w or about 2% w/w. Preferably, the viscosity is sufficiently high for the compositions to be dosed from a tube. Preferably, the viscosity of the final composition is about 1Pa.S to about 5Pa.S, more preferred about 1.8 Pa.S to about 4 Pa.S.

In embodiments comprising an acidic gelling agent, such as Carbomer(s), the compositions further comprise a basic agent. The basic agent initiates the gelling of the acidic gelling agents, such as the Carbomer gelling agent, which is a polyacrylic acid. The basic agent can be present in a concentration sufficient to adjust the pH to about 6 to about 8.5, preferably about 7.3 to about 8.2. The basic agent can be an aliphatic amine, e.g. primary, secondary and tertiary alkanolamine and primary, secondary and tertiary alkylamines. For example, monoethanolamine, diethanolamine, diisopropanolamine, triethanolamine, triisopropanolamine, dimethylamine, diethylamine, trimethylamine, triethylamine can be used. In alternative embodiments, the basic agent is an inorganic basic agent. In one embodiment, the basic agent is NaOH, KOH or ammonia solution. The ammonia solution can for example be 10% or 30% ammonia solution.

The compositions can optionally comprise further ingredients, such as perfumes, chelating agents, preservatives, stabilisers, antioxidants, colorants, or the like.

In some embodiments, the compositions disclosed herein are substantially free from permeation enhancers. Typically, permeation enhancers are included in compositions to increase cumulative permeation and/ or flux of the API. However, they can be irritating to skin. Additionally, some permeation enhancers need to be specifically highlighted on package labels. Specifically, the compositions can be free from isostearic acid and other fatty acids or acids. Permeation enhancers can yield cloudy, hazy or milky compositions and therefore are not preferred. Specifically, the compositions preferably are free from fatty alcohols, fatty acids, isopropylmyristate, isopropyl palmitate and diethyl sebacate.

Preferably, the compositions disclosed herein are substantially free from additional humectants, such as glycerol. Whilst humectants are often inlcuded in topical compositions for their cooling, conditioning and hydrating properties, they can lead to long drying time of a product on the skin. Therefore they are not preferred.

Preferably, the compositions disclosed herein are substantially free from sensates. For example, the compositions can be free from peppermint oil, 1-menthol and menthol derivatives, methyl salicylate, ethyl salicylate, glycol monosalicylate or the like. Preferably, the compositions are substantially free from counter irritants. Preferably, the compositions are substantially free from inductive stimulants. Sensates, counter irritants and inductive stimulants are often incorporated to provide the "fast acting" cooling sensation that was described above as a preferred senosory attribute. However, they can also cause turbidity or breaking of formulations. They can also lead to irritiation. Furthermore, they can influence permeation of the compositions.

### Stability

As discussed above, it is important that the API is and remains solubilised. This can be determined by the absence of API crystals. Preferably the compositions are free from crystals of API upon visual inspection with a light microscope. Preferably, the compositions are homogenous upon visual inspection with the naked eye. Preferably, the compositions are free from crystals of the API over a shelf life of 12, 24 or 36 months. In some embodiments, the compositions are homogenous upon visual inspection with a light microscope with a ×10, ×40, ×100, ×400, ×800 or ×1200 magnification. In some embodiments, the compositions are homogenous upon visual inspection with a light microscope with a ×10, ×40, ×100, ×400, ×800 or ×1200 magnification after storage under conditions for accelerated stability testing at 40°C ± 2°C/ 75% ± 5% relative humidity for 6, 12, 24 or 36 months, or under conditions for long term stability testing at 30°C ± 2°C / 65% ± 5% relative humidity over 3, 6, 12, 24 or 36 months.

### Permeation

It was an objective of the present inventors to discover a composition which over 24 hours in an *in vitro* skin permeation test provides a treatment to reference ratio (T/R) of cumulative permeation with a 90% CI that between 0.8 and 1.25, relative to the reference product Voltarol Joint Pain Relief 2.32% diclofenac diethylammonium. The method of measuring the cumulative skin permeation is explained in detail in the example section. In one embodiment, the compositions herein have a cumulative over 24 hours in an *in vitro* human skin permeation test that complies with the acceptance criteria of the Draft Guideline on quality and equivalence of topical products, CHMP/QWP/708282/2018. In one embodiment the compositions have a cumulative permeation over 24 hours in an *in vitro* human skin permeation test that complies with the acceptance criteria of the Draft Guideline on quality and equivalence of topical products, CHMP/QWP/708282/2018, lines 708 to 710. In one embodiment the compositions have a cumulative permeation over 24 hours in an *in vitro* human skin permeation test, wherein the 90% confidence interval for the ratio of means of the compositions and the comparator product, Voltarol Joint Pain Relief 2.32% diclofenac diethylammonium, lies in an acceptance interval of 80.00 - 125.00 %.

In a particular embodiment the compositions have a cumulative over 24 hours in an *in vitro* human skin permeation test that complies with the acceptance criteria of the Draft Guideline on quality and equivalence of topical products, CHMP/QWP/708282/2018, lines 711 to 715. In another embodiment the compositions have a cumulative over 24 hours in an *in vitro* human skin permeation test, wherein the 90% confidence interval for the ratio of means of the compositions and the comparator product, Voltarol Joint Pain Relief 2.32% diclofenac diethylammonium, is in an acceptance interval of 69.84% - 143.19%. Wider acceptance criteria for the 90% confidence interval can be accepted in the case of high within-subject or within-donor variability.

In some embodiments, the compositions have a cumulative permeation in an *in vitro* skin permeation test, that enables bridging to the literature data of Voltarol Joint Pain Relief 2.32% diclofenac diethylammonium for a registration under the well-established use criteria, according to Annex I of Directive 2001/83/EC.

As explained above, due to the specific selection of the solvent system and the inclusion of a hydrophilic non-ionic emulsifier, the permeation of the hydroalcoholic single phase compositions can be tailored and the cumulative permeation can be reduced compared to other hydroalcoholic single phase gel compositions. This effect can be summarised as a *permeation retarding* effect. This effect will become apparent also from the examples.

### Particular embodiments

In one embodiment, there is provided a hydroalcoholic single phase gel composition comprising:
- diclofenac or a pharmaceutically acceptable salt of diclofenac,
- a gelling agent,
- aliphatic C₂-C₄ mono-alcohol in a concentration of about 5% w/w to about 20% w/w,
- aliphatic diol in a concentration of about 2.5% w/w to about 20% w/w,
- a non-ionic emulsifier, and
- water.

In one embodiment, there is provided a hydroalcoholic single phase gel composition comprising:
- diclofenac or a pharmaceutically acceptable salt of diclofenac,
- a gelling agent,
- aliphatic C₂-C₄ mono-alcohol in a concentration of about 5% w/w to about 20% w/w,
- aliphatic diol in a concentration of about 2.5% w/w to about 20% w/w,
- a non-ionic emulsifier with a hydrophilic-lipophilic balance value of about 14 to about 16, and
- water.

In one embodiment, there is provided a hydroalcoholic single phase gel composition comprising:
- diclofenac or a pharmaceutically acceptable salt of diclofenac,
- a gelling agent,
- aliphatic C₂-C₄ mono-alcohol in a concentration of about 5% w/w to about 20% w/w,
- aliphatic diol in a concentration of about 2.5% w/w to about 20% w/w,
- Macrogol Cetostearyl Ether 20, and
- water.

In one embodiment, there is provided a hydroalcoholic single phase gel composition comprising:
- diclofenac or a pharmaceutically acceptable salt of diclofenac,
- a gelling agent,
- aliphatic C₂-C₄ mono-alcohol in a concentration of about 5% w/w to about 20% w/w,
- aliphatic diol in a concentration of about 2.5% w/w to about 20% w/w,
- a non-ionic emulsifier with a hydrophilic-lipophilic balance value of about 16 to about 18, and
- water.

In one embodiment, there is provided a hydroalcoholic single phase gel composition comprising:
- diclofenac or a pharmaceutically acceptable salt of diclofenac,
- a gelling agent,
- aliphatic C₂-C₄ mono-alcohol in a concentration of about 5% w/w to about 20% w/w,
- aliphatic diol in a concentration of about 2.5% w/w to about 20% w/w,
- Polysorbate 20, and
- water.

In another embodiment, there is provided a hydroalcoholic single phase gel composition for topical delivery of diclofenac, comprising:
- diclofenac or a pharmaceutically acceptable salt of diclofenac,
- a gelling agent,
- aliphatic C₂-C₄ mono-alcohol in a concentration of about 5% w/w to about 20% w/w,
- aliphatic diol in a concentration of about 2.5% w/w to about 20% w/w,
- water,
   wherein the composition does is substantialla free of a lipophilic phase.

In another embodiment, there is provided a hydroalcoholic single phase gel composition for topical delivery of diclofenac, comprising:
- diclofenac or a pharmaceutically acceptable salt of diclofenac,
- a gelling agent,
- aliphatic C₂-C₄ mono-alcohol in a concentration of about 5% w/w to about 20% w/w,
- aliphatic diol in a concentration of about 2.5% w/w to about 20% w/w,
- at least one non-ionic emulsifier, in a concentration of about 0.5% w/w to about 4% w/w, and
- water.

In another embodiment, there is provided a hydroalcoholic single phase gel composition for topical delivery of diclofenac, comprising:
- diclofenac or a pharmaceutically acceptable salt of diclofenac,
- a gelling agent,
- aliphatic C₂-C₄ mono-alcohol in a concentration of about 5% w/w to about 20% w/w,
- a glycol in a concentration of about 2.5% w/w to about 20% w/w,
- a non-ionic emulsifier, in a concentration of about 0.5% w/w to about 4% w/w, and
- water.

In another embodiment, there is provided a hydroalcoholic single phase gel composition for topical delivery of diclofenac, comprising:
- diclofenac or a pharmaceutically acceptable salt of diclofenac,
- a gelling agent,
- isopropanol, ethanol, or combinations thereof,
- propylene glycol,
- a non-ionic emulsifier, and
- water.

In another embodiment, there is provided a hydroalcoholic single phase gel composition for topical delivery of diclofenac, comprising:
- diclofenac or a pharmaceutically acceptable salt of diclofenac, in a concentration of about 1.5% w/w to about 2.5% w/w diclofenac sodium equivalent,
- a Carbomer gelling agent,
- an aliphatic C₂-C₄-mono-alcohol, in a concentration of about 5% w/w to about 20% w/w,
- a C₂-C₄-polyalcohol, in a concentration of about 2.5% w/w to about 20% w/w,
- a non-ionic emulsifier, in a concentration of about 0.1% w/w to about 5% w/w, and
- water, and
- a basic agent to adjust the pH of the total composition to about 6.5 to about 8.5.

In another embodiment, there is provided a composition consisting essentially of:
- diclofenac or a pharmaceutically acceptable salt of diclofenac,
- a gelling agent,
- an aliphatic C₂-C₄-mono-alcohol,
- an aliphatic diol,
- a non-ionic emulsifier,
- water, and
- a basic agent to adjust the pH of the total composition to a pH of about 6.5 to about 8.5, and optionally perfumes, chelating agents, preservatives, stabilisers, antioxidants, and/ or colorants.

In another embodiment, there is provided a composition consisting essentially of:
- diclofenac or a pharmaceutically acceptable salt of diclofenac,
- a gelling agent,
- an aliphatic C₂-C₄-mono-alcohol,
- an aliphatic diol,
- a first non-ionic emulsifier with a hydrophilic-lipophilic balance value of about 14 to about 16,
- a second non-ionic emulsifier with a hydrophilic-lipophilic balance value of about 16 to about 18,
- water, and
- a basic agent to adjust the pH of the total composition to a pH of about 6.5 to about 8.5, and optionally perfumes, chelating agents, preservatives, stabilisers, antioxidants, and/ or colorants.

Preferably, the above compositions are substantially free of anionic emulsifier. In some embodiments, the above compositions are substantially free of anionic emulsifier and substantially free of cationic emulsifier.

### METHODS OF MANUFACTURE

The compositions can be manufactured as follows:
- In a first vessel, dispersing gelling agent in a first portion of water, until fully dispersed;
- If required to initiate gelling of the gelling agent, adding a basic agent to the gelling agent dispersion, to obtain a gel;
- In a second vessel, combining a second portion of water, aliphatic mono-alcohol, aliphatic diol and non-ionic emulsifier(s);
- Dispersing diclofenac or pharmaceutically acceptable salt of diclofenac in the mixture in the second vessel, to obtain an API solution;
- Combining the gel and the API solution under homogenisation.

### USE

The compositions disclosed herein are suitable for use in the treatment of:
- Pain, inflammation and swelling in:
   - Soft-tissue injuries: trauma of the tendons, ligaments, muscles and joints, e.g. due to sprains, strains, bruises and backache (sports injuries)
   - Localised forms of soft tissue rheumatism: tendonitis (e.g. tennis elbow), bursitis, shoulder-hand syndrome and periarthropathy; and
- Pain of non-serious arthritis of the knee or fingers.

### PHARMACEUTICAL COMPOSITIONS/ROUTES OF ADMINISTRATION/DOSAGES

The compositions can be administered to mammals suffering from joint pain, osteoarthritis, muscle pain, back pain and/ or inflammation. Preferably, the compositions are for the treatment of humans. The compositions are useful in the treatment of pain, inflammation and swelling in soft-tissue injuries such as trauma of the tendons, ligaments, muscles and joints, e.g. due to sprains, strains, bruises and backache (sports injuries), and/ or localised forms of soft tissue rheumatism, such as tendonitis (e.g. tennis elbow), bursitis, shoulder-hand syndrome and periarthropathy.

The compositions are also useful in the treatment of pain of non-serious arthritis of the knee or fingers. They can be applied onto the skin covering the concerned body part. They may be rubbed in until the skin feels dry. They may be applied 2-4 times per day. The methods of treatment can comprise administering to a subject in need thereof a pharmaceutically effective amount of the compositions disclosed herein. The method can comprise administering the composition to skin that covers a body part suffering from or causing one or more of pain, inflammation and swelling in soft-tissue injuries such as trauma of the tendons, ligaments, muscles and joints, e.g. due to sprains, strains, bruises and backache (sports injuries), and/ or localised forms of soft tissue rheumatism, such as tendonitis (e.g. tennis elbow), bursitis, shoulder-hand syndrome and periarthropathy, or pain of non-serious arthritis. The compositions can be provided in a package comprising a means for dosing. The compositions may be provided in a container comprising an applicator for direct application onto the skin.

Embodiments described herein can be understood more readily by reference to the following detailed description, examples, and figures. Elements, apparatus, and methods described herein, however, are not limited to the specific embodiments presented in the detailed description, examples, and figures. It should be recognised that the exemplary embodiments herein are merely illustrative of the principles of the present invention. Numerous modifications and adaptations will be readily apparent to those of skill in the art without departing from the spirit and scope of the invention.

In addition, all ranges disclosed herein are to be understood to encompass any and all subranges subsumed therein. For example, a stated range of "1.0 to 10.0" should be considered to include any and all subranges beginning with a minimum value of 1.0 or more and ending with a maximum value of 10.0 or less, e.g., 1.0 to 5.3, or 4.7 to 10.0, or 3.6 to 7.9.

All ranges disclosed herein are also to be considered to include the end points of the range, unless expressly stated otherwise. For example, a range of "between 5 and 10" or "5 to 10" or "5-10" should generally be considered to include the end points 5 and 10.

It is further to be understood that the feature or features of one embodiment may generally be applied to other embodiments, even though not specifically described or illustrated in such other embodiments, unless expressly prohibited by this disclosure or the nature of the relevant embodiments. Likewise, compositions and methods described herein can include any combination of features and/ or steps described herein not inconsistent with the objectives of the present disclosure. Numerous modifications and/ or adaptations of the compositions and methods described herein will be readily apparent to those skilled in the art without departing from the present subject matter. Unless indicated otherwise, concentrations are given in %(w/w).

Unless indicated otherwise, concentrations are given in %(w/w).

The term "about" in relation to a numerical value x means, for example, x±10%, x±5%, x±4%, x±3%, x±2%, x±1%.

pH, viscosity and HLB are typically measured at 25°C.

### EXAMPLES

Hereinafter, the present invention will be described in more detail with reference to examples, but the present invention is not limited by these examples.

### Example 1: Comparison of cumulative skin permeation at 24hrs in emulgels and hydroalcoholic gels

An in vitro skin permeation study was conducted in a preclinical laboratory. Skin permeation studies can be used to determine the qualitative permeation characteristics of a formulation through, e.g., human skin. They can also be used for direct quantitative comparison the permeation and flux of two given formulations. In Example 1,cumulative permeation of the hydroalcoholic test formulations was compared to cumulative permeation of a reference product, which was an emulgel. Five test formulations and the reference product were each applied to human donor abdominal skin at timepoint 0hrs in a dose of 10mg/cm². Each formulation was tested on five skin samples from different donors on a Franz diffusion cell. The reference product for comparison was Voltaren Emulgel 1.16% (m/m) Diclofenac diethylammonium. The composition of the test formulations and of the reference product Voltaren Emulgel 1.16% (m/m) are listed in Table 1:

**Table 1: Composition of the Reference Product and the test formulations in Example 1**

| | Referenc e product in Example 1) | Test 1 | Test 2 | Test 3 | Test 4 | Test 5 |
|---|---|---|---|---|---|---|
| Ingredient | Concentration [% w/w] | | | | | |
| Diclofenac Diethylamine | 1.16 | 1.16 | 1.16 | 1.16 | 1.16 | 1.16 |
| Carbomer 974P | 1.20 | 1.20 | 1.20 | 1.20 | 1.20 | 1.20 |
| Isopropyl alcohol | 20.00 | 20.00 | 7.50 | 10.00 | 12.50 | 12.50 |
| Propylene glycol | 5.00 | 5.00 | 12.50 | 5.00 | 5.00 | 7.50 |
| Diethylamine | 0.90 | 0.90 | 0.90 | 0.90 | 0.90 | 0.90 |
| Perfume cream 45 | 0.10 | 0.10 | 0.10 | 0.10 | 0.10 | 0.10 |
| Cocoyl caprylocaprate | 2.50 | / | / | / | / | / |
| Paraffin, liquid | 2.50 | / | / | / | / | / |
| Cetomacrogol 1000 | 2.00 | / | / | / | / | / |
| Purified Water | 64.64 | 71.64 | 76.64 | 81.64 | 79.14 | 76.64 |

The concentration of diclofenac in the receptor fluid of the Franz cell was measured at timepoints t=0hrs, 4hrs, 8hrs and 24hrs, and the study endpoint was the cumulative amount of diclofenac permeated at 24hrs.

The results of the study are given in Table 2.

**Table 2: Results of the in vitro skin permeation study of Example 1**

| | Reference Product | Test 1 | Test 2 | Test 3 | Test 4 | Test 5 |
|---|---|---|---|---|---|---|
| N | 5 | 5 | 4 | 5 | 5 | 5 |
| Min | 1632 | 2071 | 2929 | 3591 | 2825 | 4923 |
| Median | 4098 | 9101 | 5577 | 6941 | 3869 | 8300 |
| Max | 5176 | 27165 | 20689 | 17993 | 28338 | 20828 |
| Geometric mean | 3419 | 7900 | 6589 | 8678 | 5757 | 9445 |
| CV (%) | 49.11 | 148.14 | 98.10 | 76.46 | 122.45 | 66.16 |
| Ratio vs. reference product [90% CI] | n.a. | 2.31 [0.96; 5.58] | 2.08 [1.21; 3.58] | 2.54 [1.85; 3.47] | 1.68 [0.82; 3.45] | 2.76 [1.87; 4.09] |

Surprisingly, the cumulative permeation of all test formulations Test 1 to Test 5 was well above the cumulative permeation of the Reference Product. Whilst the concentration of the active ingredient was the same in all tested compositions, the hydroalcoholic formulations Test 1 to Test 5 showed a cumulative permeation at 24hrs that was between about 1.7x (Test 4) to almost 3x (Test 5) as high as the cumulative permeation of the Reference Product. This led the present inventors to the conclusion that the absence of a dispersed lipophilic phase leads to a higher cumulative permeation at 24hrs.

### Example 2: Comparison of cumulative skin permeation at 24hrs in emulgels and hydroalcoholic gels

In a cumulative skin permeation study in a 3ml permeation cell, cumulative permeation through a skin sample from a human donor was studied. Five different compositions were tested in six replicates. Cumulative permeation was measured at 4 hours (hrs) and at 24 hrs after application of a dose. However, cumulative permeation at 4 hrs was below the measurement threshold in this experiment and thus is not reported for Example 2. All formulations were applied in a dose of 6 microlitres onto skin sample of a defined surface area. The following formulations were studied:

**Table 3: Compositions of the comparator and test compositions and cumulative permeation results**

| **Formulations** | | | | | |
|---|---|---|---|---|---|
| | **Voltaren Arthritis Pain** | **F072** | **F077** | **F078** | **F079** |
| Diclofenac sodium | 1 | 2 | 2 | 2 | 2 |
| Carbomer | 1.6 | 2.6 | 2.6 | 2.6 | 2.6 |
| Isopropanol | 20.2 | 16 | 16 | 16 | 16 |
| Propylene glycol | 5 | 16 | 16 | 16 | 16 |
| Kolliphor CS 20 | 2 | - | 2 | - | 2 |
| Kolliphor PS 20 | - | - | - | 2 | 2 |
| Water | 61.6 | 49.4 | 47.4 | 47.7 | 45.4 |
| STOCK 1¹ | - | 1 | 1 | 1 | 1 |
| STOCK 2² | - | 13 | 13 | 13 | 13 |
| Cocoyl caprylocaprate | 2.5 | - | - | - | - |
| Mineral oil | 2.5 | - | - | - | - |
| Ammonia 10% solution | 3.5 | - | - | - | - |
| Perfume cream | 0.1 | - | - | - | - |

| **Cumulative permeation at 24 hrs [µg/cm²]** | | | | | |
|---|---|---|---|---|---|
| | 0.94 | 4.78 | 1.92 | 2.94 | 1.41 |

Voltaren Arthritis Pain is an emulgel composition, whilst the test compositions F072, F077, F078 and F079 were hydroalcoholic gel compositions comprising no non-ionic emulsifier (F072), one non-ionic emulsifier (F077 and F078), or a combination of two non-ionic emulsifiers (F079). Voltaren Arthritis Pain was used as a comparator, as it was intended to study the difference between emulgels and hydroalcoholic gels, and because the product was readily available for purchase.

The results are summarised in the table above, and are visualised in Figure 2. All test compositions showed higher cumulative permeation at 24 hrs than the test composition. It had been
¹ Stock solutions were used for simplified formulation in the experiments. STOCK 1 contained water (95.33% w/w), sodium phosphate dibasic (4.00% w/w) and sodium phosphate monobasic (0.67% w/w).
² Stock solutions were used for simplified formulation in the experiments. STOCK 2 contained water (80% w/w) and ammonia strong solution (20.00% w/w). expected that the cumulative permeation from the test compositions was higher than that of the comparator, because the test compositions contained more diclofenac sodium (2% w/w instead of 1% w/w). However, the magnitude of the difference was surprisingly high and not expected. Interestingly, formulation F072, which contained the same excipient types as the comparator, except for the lipophilic phase (Cocoyl caprylocaprate and mineral oil) and emulsifier (Kolliphor CS 20), showed a cumulative permeation at 24hrs of almost five times higher than the comparator. Unexpectedly, this significant increase of cumulative permeation at 24 hrs could be reduced by incorporation of a non-ionic emulsifier. Contrary to the teachings in the art, non-ionic emulsifier can thus have a permeation retarding effect. Comparing the cumulative permeation at 24hrs of all test formulations, Kolliphor PS 20 and Kolliphor CS 20 both have a permeation retarding effect, with the permeation retarding effect of Kolliphor CS 20 being more pronounced. Formulation F079, which contained a combination of Kolliphor CS20 and Kolliphor PS 20, at a total concentration of 4% w/w, showed the lowest cumulative permeation at 24hrs of all test formulations.

### Example 3: Comparison of cumulative skin permeation at 24hrs in emulgels and hydroalcoholic gels comprising 2% w/w diclofenac epolamine

To test the hypothesis that non-ionic emulsifiers showed the same effect in compositions comprising different salts of diclofenac, especially with diclofenac salts with organic counterions, the experiment of Example 1 was repeated with test compositions comprising diclofenac epolamine. The comparator was the same as in Example 1. If not specified otherwise, the experimental setup was the same as in Example 1.

**Table 4: Compositions of the comparator and test formulations and cumulative permeation results**

| **Formulations** | | | | | |
|---|---|---|---|---|---|
| | **Voltaren Arthritis Pain** | **DiEp-F004** | **DiEp-F005** | **DiEp-F006** | **DiEp-F007** |
| Diclofenac | 1 (sodium) | 2 (epolamine) | 2 (epolamine) | 2 (epolamine) | 2 (epolamine) |
| Carbomer | 1.6 | 2.6 | 2.6 | 2.6 | 2.6 |
| Isopropanol | 20.2 | 20 | 20 | 20 | 20 |
| Propylene glycol | 5 | 20 | 20 | 20 | 20 |
| Kolliphor CS 20 | 2 | - | 2 | - | 2 |
| Kolliphor PS 20 | - | - | - | 2 | 2 |
| Water | 61.6 | 41.4 | 39.4 | 39.4 | 37.4 |
| STOCK 1³ | - | 1 | 1 | 1 | 1 |
| STOCK 2⁴ | - | 13 | 13 | 13 | 13 |
| Cocoyl caprylocaprate | 2.5 | - | - | - | - |
| Mineral oil | 2.5 | - | - | - | - |
| Ammonia 10% solution | 3.5 | - | - | - | - |
| Perfume cream | 0.1 | - | - | - | - |

| **Cumulative permeation at 4 hrs [µg/cm²]** | | | | | |
|---|---|---|---|---|---|
| | 8.56 | 9.31 | 3.62 | 2.69 | 3.86 |

| **Cumulative permeation at 24 hrs [µg/cm²]** | | | | | |
|---|---|---|---|---|---|
| | 45.84 | 101.34 | 32.12 | 43.38 | 33.44 |

The results are summarised in the table above, and are visualised in Figure 3. Interestingly, only formulation DiEp-F004, the test composition comprising no lipophilic phase and no emulsifier, showed a significantly higher cumulative permeation than the comparator. Surprisingly, all test compositions comprising one (DiEp-F005 and DiEp-F006) non-ionic emulsifier or two non-ionic emulsifiers (DiEp-F007) showed a cumulative permeation at 24hrs even lower than the cumulative permeation of the comparator. It therefore seems that a permeation retarding effect of non-ionic emulsifiers is even more pronounced in compositions comprising diclofenac epolamine than in compositions comprising diclofenac sodium (Example 2). Interestingly, formulations DiEp-F005, Di-Ep-F006 and DiEp-F007 showed very similar cumulative permeation at 24hrs. In formulations comprising diclofenac epolamine, Kolliphor CS 20 and Kolliphor PS 20 appear to have a similar permeation retarding effect. When used in combination at a total concentration of about 4% w/w (DiEp-F007), the cumulative permeation at 24hrs was further lowered.

### Example 4: Comparison of cumulative skin permeation at 24hrs in emulgels and hydroalcoholic gels comprising 2% w/w diclofenac sodium and different concentrations of non-ionic emulsifier in non-GLP comparative skin permeation study in different posologies

Hydroalcoholic compositions comprising 2% w/w diclofenac sodium and different levels of non-ionic emulsifiers were studied. At the same time, concentrations of isopropanol and propylene
³ Stock solutions were used for simplified formulation in the experiments. STOCK 1 contained water (95.33% w/w), sodium phosphate dibasic (4.00% w/w) and sodium phosphate monobasic (0.67% w/w).
⁴ Stock solutions were used for simplified formulation in the experiments. STOCK 2 contained water (80% w/w) and ammonia strong solution (20.00% w/w). glycol were varied to study the influence of these ingredients on cumulative permeation. The comparator was Voltarol Joint Pain Relief, also known in some markets under Voltaren 2.32% diclofenac diethylamine (DDEA) Emulgel.

Split-thickness abdominal human skin samples (200-400 µm) with transepithelial electrical resistance (TEER) values >7.7 kΩ were mounted in static diffusion cells (exposure area = 0.64 cm²) and kept under controlled temperature conditions (32 ± 1°C). Each study formulation was applied in duplicate to skin samples from 6 donors (i.e. total skin samples per formulation = 12 = N). Two experiments were conducted to evaluate the impact of dosing once (*t* = 0 h) and four times (*t* = 0, 4, 8, 12 h). The receptor fluid was phosphate buffered saline with 5% bovine serum albumin. Receptor fluid samples were collected at 0, 2, 4, 8, 16 and 24 hrs post first dose and analysed for diclofenac content by LC-MS/MS. Cumulative absorption of diclofenac in the receptor fluid was calculated for each test formulation and for both dosing intervals. Cumulative absorption of diclofenac at 24 hrs post first dose was analysed. The half-value of the limit of quantification (LOQ) was used for any post-baseline samples with a concentration below the LOQ. The statistical model used was analysis of variance (ANOVA) on log-transformed geometric mean values and included formulation and donor as fixed effects.

**Table 5: Compositions of the comparator and test formulations and cumulative permeation results**

| | Voltarol Joint Pain Relief | **Test 9** | **Test 10** | **Test 11** | **Test 12** | **Test 13** | **Test 14** |
|---|---|---|---|---|---|---|---|
| Diclofenac sodium/ DEA | 2.32⁵ | 2.00 | 2.00 | 2.00 | 2.00 | 2.00 | 2.00 |
| Isopropanol | 17.50 | 15.00 | 12.50 | 17.50 | 17.50 | 12.50 | 12.50 |
| Propylene glycol | 5.00 | 15.00 | 10.00 | 5.00 | 5.00 | 12.50 | 12.50 |
| Kolliphor CS 20 | 2.00 | 1.00 | 2.00 | 2.00 | 2.00 | 2.00 | 2.00 |
| Kolliphor PS 20 | / | 2.00 | 1.00 | / | 1.00 | / | 1.00 |
| Carbopol 980 | 1-2⁶ | 2.00 | 2.00 | 2.00 | 2.00 | 2.00 | 2.00 |
| Cocoyl caprylocaprate | 2.50 | - | - | - | - | - | - |
| Paraffin, liquid | 2.50 | - | - | - | - | - | - |
| Ammonia 25% solution | / | 2.00 | 2.00 | 2.00 | 2.00 | 2.00 | 2.00 |
| Perfume cream | 0.1 | - | - | - | - | - | - |
| Oleyl alcohol | 0.75 | - | - | - | - | - | - |
| Butylhydroxytoluene | 0.02 | - | - | - | - | - | - |
| Purified Water | Ad. 100 | 61.00 | 68.50 | 69.50 | 68.50 | 67.00 | 66.00 |

⁵ Voltarol Joint Pain Relief contains 2.32% w/w diclofenac diethylammonium (DEA) salt, i.e. the equivalent to 2.00% w/w diclofenac sodium salt
⁶ Voltarol Joint Pain Relief specifies a range for the gelling agent, basic agent and water, to account for variations between batches.

**Table 6: Results of Example 4:**

| | Voltarol Joint Pain Relief | **Test 9** | **Test 10** | **Test 11** | **Test 12** | **Test 13** | **Test 14** |
|---|---|---|---|---|---|---|---|
| N | 12 | 12 | 12 | 12 | 12 | 12 | 12 |
| Min | 740.89 | 1611.00 | 1000.32 | 976.41 | 517.68 | 3291.55 | 826.81 |
| Median | 2509.97 | 6474.24 | 4086.05 | 1898.43 | 1780.61 | 7172.18 | 3563.54 |
| Max | 11919.33 | 12918.43 | 8192.56 | 6908.74 | 6358.23 | 13393.54 | 7970.67 |
| Mean | 4289.15 | 6454.94 | 4048.52 | 2851.60 | 2236.37 | 6957.14 | 3943.36 |
| Geometric mean | 2879.17 | 5316.90 | 3246.02 | 2334.98 | 1678.65 | 6296.97 | 3336.95 |
| Geo-mean CV (%) | 125.69 | 79.85 | 86.29 | 71.94 | 95.48 | 49.89 | 72.17 |
| Geometric LS mean Test | 2879.17 | 5316.90 | 3246.02 | 2334.98 | 1678.65 | 6296.97 | 3336.95 |
| Ratio | 1 | 1.85 | 1.13 | 0.81 | 0.58 | 2.19 | 1.16 |
| 90% CI | - | [1.33, 2.56] | [0.83, 1.52] | [0.61, 1.08] | [0.45, 0.76] | [1.59, 3.01] | [0.82, 1.64] |
| 60% CI | - | [1.57, 2.17] | [0.97, 1.31] | [0.70, 0.94] | [0.51, 0.66] | [1.87, 2.56] | [0.98, 1.37] |

The results are summarised in the table above.

In Example 4, it was observed that the cumulative permeation of the compositions test 10 was most similar to the cumulative permeation of the reference product. For Test 10, Test 11 and Test 14, the 60% CI of the geometric least squares mean ratio of cumulative absorption of diclofenac in the receptor fluid 24 h post dose was entirely within 0.67-1.50. Therefore, these test formulations are considered to have permeation characteristics that are most similar to the comparator. Comparing the results of Test 9, Test 13 and Test 14 on the one hand, each comprising above 10% w/w propylene glycol, with the results of Test 10, Test 11 and Test 12, each comprising less than 10% w/w propylene glycol, it is apparent that the higher concentration of propylene glycol results in higher cumulative permeation. Comparing only Test 14 and Test 13: Test 13 and Test 14 have the same composition, except that Test 13 only comprises 2.00% w/w Kolliphor CS20, and Test 14 additionally comprises 1.00% w/w Kolliphor PS 20 (on the account of water). The addition of the second non-ionic emulsifier reduces the cumulative permeation, even to a degree that Test 14, although it comprises more than 10% w/w propylene glycol, has a cumulative permeation with the 60% CI of the geometric least squares mean ratio of cumulative absorption of diclofenac in the receptor fluid 24 h post dose being entirely within 0.67-1.50. Test 12, the test composition comprising two non-ionic emulsifiers and only 5% w/w propylene glycol, had the lowest cumulative permeation.

## Claims

1. A hydroalcoholic single phase gel composition for topical delivery of diclofenac, comprising:
- diclofenac or a pharmaceutically acceptable salt of diclofenac,
- a gelling agent,
- aliphatic C₂-C₄ mono-alcohol in a concentration of about 5% w/w to about 20% w/w,
- aliphatic diol in a concentration of about 2.5% w/w to about 20% w/w,
- a non-ionic emulsifier, and
- water.

2. A composition according to claim 1, comprising diclofenac or a pharmaceutically acceptable salt of diclofenac, in a total concentration of about 1.5% w/w to about 2.5% w/w diclofenac sodium equivalent.

3. A composition according to claim 1 or 2, comprising at least two different non-ionic emulsifiers.

4. A composition according to any of the preceding claims, comprising a non-ionic emulsifier with a hydrophilic-lipophilic balance value of about 13 to about 17, about 14 to about 16, or about 15.

5. A composition according to any of the preceding claims, comprising a non-ionic emulsifier with a hydrophilic-lipophilic balance value of about 15 to about 19, about 16 to about 18, or about 17.

6. A composition according to any of the preceding claims, comprising a non-ionic emulsifier selected from the group consisting of Macrogol Cetostearyl Ether 20, Polysorbate 60, Polysorbate 80, Isosteareth 20, PEG-60 Almond Glycerides, PEG-20 Methyl Glucose Sesquistearate, Oleth-20, Steareth-20, and combinations of two or more thereof.

7. A composition according to any of the preceding claims, comprising a macrogol cetostearyl ether.

8. A composition according to any of the preceding claims, comprising a non-ionic emulsifier selected from the group of Polysorbate 20, Laureth-20, and combinations thereof.

9. A composition according to any of the preceding claims, comprising Polysorbate 20.

10. A composition according to any of the preceding claims, comprising a non-ionic emulsifier comprising a 2-isopropyl-tetrahydrofuran moiety comprising one or more oligo(ethylene oxide) ester group, wherein all ethylene oxide units of the oligo(ethylene oxide) ester groups in combination comprise 10 to 30, 14 to 24, or 20 carbon atoms.

11. A composition according to any of the preceding claims, comprising a first non-ionic emulsifier with a hydrophilic-lipophilic balance value of about 14 to about 16, and a second non-ionic emulsifier with a hydrophilic-lipophilic balance value of about 16 to about 18.

12. A composition according to any of the preceding claims, comprising a first non-ionic emulsifier which is a macrogol cetostearyl ether and a second non-ionic emulsifier which is a polysorbate.

13. A composition according to any of the preceding claims, comprising non-ionic emulsifier in a total concentration of about 0.1% w/w to about 5% w/w, about 1% w/w to about 3.5% w/w or about 1.5% w/w to about 3% w/w.

14. A composition according to any of the preceding claims, wherein the non-ionic emulsifier retards permeation of diclofenac.

15. A composition according to any of the preceding claims, having a cumulative permeation in an *in vitro* skin permeation test, that enables to rely on literature data of Voltarol Joint Pain Relief 2.32% diclofenac diethylammonium, for a registration under the well-established use criteria, according to Annex I of Directive 2001/83/EC.

16. A composition according to any of the preceding claims, for use in a method of treatment of pain of non-serious arthritis of the knee or fingers.

17. Use of a non-ionic emulsifier to reduce cumulative permeation of diclofenac through the skin over a period of 6 hours, 9 hours, 12 hours or 24 hours, from a hydroalcoholic single phase gel for topical delivery of diclofenac.
